(19) Europäisches Patentamt
European Patent Office
Office européen des brevets

(11) Numéro de publication : **0 057 045**
**B1**

(12) **FASCICULE DE BREVET EUROPÉEN**

(45) Date de publication du fascicule du brevet : **05.09.84**

(51) Int. Cl.³ : **C 07 D307/32**, C 07 C 59/74, C 07 F 9/54// C07D307/93

(21) Numéro de dépôt : **82200370.3**

(22) Date de dépôt : **03.06.80**

(60) Numéro de publication de la demande initiale en application de l'article 76 CBE : **0023849**

(54) **Nouveaux dérivés du 5-hydroxy tétrahydrofuran 2-one.**

(30) Priorité : **06.06.79 FR 7914425**

(43) Date de publication de la demande : **04.08.82 Bulletin 82/31**

(45) Mention de la délivrance du brevet : **05.09.84 Bulletin 84/36**

(84) Etats contractants désignés : **AT BE CH DE GB IT LI LU NL SE**

(56) Documents cités :
**EP-A- 0 024 241**
**EP-A- 0 037 327**
**JOURNAL OF ORGANIC CHEMISTRY, vol. 39, no. 1, 11 janvier 1974 WASHINGTON (US) K. OHGA et al.: "Photoinduced addition of isopropyl alcohol to alpha, beta-unsaturated lactones" pages 106-108**

(73) Titulaire : **ROUSSEL-UCLAF**
**35, boulevard des Invalides**
**F-75007 Paris (FR)**

(72) Inventeur : **Martel, Jacques**
**15, rue Douvillez**
**F-93140 Bondy (FR)**
Inventeur : **Tessier, Jean**
**30, rue Jean Moulin**
**F-94300 Vincennes (FR)**
Inventeur : **Demoute, Jean-Pierre**
**249 bis, rue de Rosny**
**F-93100 Montreuil-sous-Bois (FR)**

(74) Mandataire : **Vieillefosse, Jean-Claude et al**
**ROUSSEL-UCLAF Boîte postale no. 9 111, route de Noisy**
**F-93230 Romainville (FR)**

**EP 0 057 045 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention a pour objet de nouveaux dérivés du 5-hydroxy tétrahydrofuranne 2-one, leur procédé de préparation et leur application à la préparation de l'acide 3-formyl 4-méthyl pent-3-en-1-oique.

L'invention a pour objet les composés de formule générale IV :

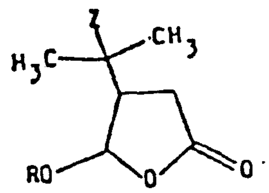

(IV)

dans laquelle Z représente un groupement électroattracteur et notamment un groupement hydroxy, un groupement nitro, un groupement arylsulfonyle ou un groupement halogéno triaryle phosphonio et R représente le reste d'un alcool R—OH.

L'invention a plus précisément pour objet les composés de formule IV, caractérisés en ce que le groupement Z est un groupement $NO_2$.

L'invention a également pour objet les composés de formule IV, caractérisés en ce que le groupement Z est un groupement hydroxyle.

L'invention a également pour objet les composés de formule IV, caractérisés en ce que le groupement Z est un groupement arylesulfonyle.

Le groupement aryle du groupement arylsulfonyle est notamment un groupement paratolyle.

L'invention a également pour objet les composés de formule IV, caractérisés en ce que le groupement Z est un groupement halogénotriarylphosphonio.

Le groupement halogénotriarylphosphonio est notamment le radical iodotriphényl phosphonio.

L'invention a également pour objet un procédé de préparation des composés de formule IV, caractérisé en ce que l'on fait réagir un dérivé de formule II :

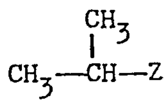

(II)

avec un dérivé de formule III :

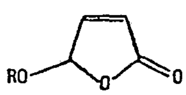

(III)

dans lesquelles R et Z ont les mêmes significations que précédemment pour obtenir le composé de formule IV correspondant désiré.

L'invention a plus précisément pour objet un procédé tel que décrit précédemment, caractérisé en ce que le groupement Z est un groupement $NO_2$ et en ce que l'on fait réagir les composés II et III en présence d'une base choisie dans le groupe constitué par les amines secondaires, les amines tertiaires, les hydroxydes d'ammonium quaternaires et les carbonates alcalins.

Comme amine tertiaire en présence de laquelle on fait réagir les composés II et III, on utilise avantageusement la triéthylamine.

L'invention a également pour objet un procédé de préparation, caractérisé en ce que le groupement Z est un groupement hydroxyle et en ce que l'on fait réagir les composés II et III sous irradiation ou en présence d'un promoteur radicalaire. Comme promoteur radicalaire, on utilise avantageusement le péroxyde de benzoyle, l'azo bis iso butyronitrile, le péroxyde de diterbutyle, le perbenzoate de tertbutyle, le péroxyde de dilauryle.

L'invention a également pour objet un procédé tel que défini précédemment, caractérisé en ce que le groupement Z est un groupement aryl sulfonyle et en ce que l'on fait réagir les composés II et III en présence d'une base forte au sein d'un solvant organique.

L'invention a également pour objet un procédé tel que défini précédemment, caractérisé en ce que le groupement Z est un groupement halogéno triaryl phosphonio et en ce que l'on fait réagir les composés II et III en présence d'une base forte au sein d'un solvant organique.

L'invention a également pour objet un procédé de préparation tel que défini précédemment, caractérisé en ce que la base forte est choisie dans le groupe constitué par les alkyl lithiens, les amidures alcalins, les hydrures alcalins, les alcoolates alcalins et en ce que le solvant utilisé est choisi dans le groupe constitué par le tétrahydrofurane, le diméthylsulfoxyde, le diméthoxyéthane, le diméthylformamide, l'hexaméthylphosphoramide, les hydrocarbures aromatiques et des cycloalcanes.

Lorsque Z est un groupement aryl sulfonyle ou un groupement halogéno triaryl phosphonio, la réaction entre les composés II et III est effectuée avantageusement en présence de butyllithium au sein d'un mélange de tétrahydrofuranne et de cyclohexane. L'invention a également pour objet l'application des composés de formule générale IV à la préparation de l'acide 3-formyl 4-méthyl pent-3-èn 1-oïque caractérisé en ce que l'on soumet un composé de formule générale IV :

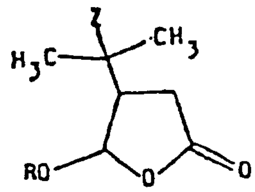

(IV)

dans laquelle R et Z conservent les significations de la revendication 1, à l'action d'un agent basique pour obtenir l'acide 3-formyl 4-méthyl pent-3-èn 1-oïque.

Selon l'application de l'invention, l'agent basique que l'on fait réagir sur les composés IV est notamment le carbonate de sodium utilisé en milieu hydrométhanolique.

L'acide 3-formyl 4-méthyl pent-3-en-1-oique est un produit décrit et revendiqué dans la demande de brevet européen n° 82 200370.3 publiée sous le n° 0023849.

Ce produit peut être utilisé pour la préparation de dérivés cyclopropaniques substitués de formule V :

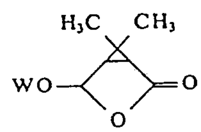

(V)

dans laquelle W représente un atome d'hydrogène ou un reste $R_1$ d'un alcool $R_1OH$ éventuellement chiral, caractérisée en ce que l'on fait réagir en milieu anhydre l'acide 3-formyl 4-méthyl pent-3-èn 1-oïque avec un hydracide HX, dans lequel X représente un atome d'halogène, en présence d'un halogénure de lithium LiX, au sein d'un solvant organique, pour obtenir un composé de formule VI :

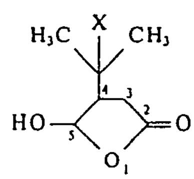

(VI)

que l'on fait réagir soit avec un alcool $R_1OH$ achiral pour obtenir le composé VII racémique :

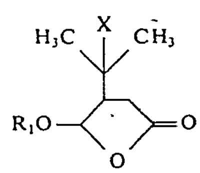

(VII)

qui, comme de dérivé VI, présente une relation trans entre les substituants en 4 et 5 de la tétrahydrofuranone, soit avec un alcool $R_1OH$ chiral, le composé VII étant alors obtenu sous forme d'un mélange des deux diastéréoisomères prévisibles, mélange que l'on peut séparer en ses constituants par des procédés physiques, fait réagir le composé de formule VII, sous forme racémique lorsque $R_1$ est achiral, ou sous forme optiquement active lorsque $R_1$ est chiral, le composé VII étant, dans ce dernier cas, à l'état de l'un ou l'autre des diastéréoisomères, avec un agent basique, pour obtenir le composé bicyclique de formule V' :

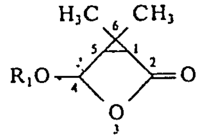

(V')

sous forme racémique, ou sous l'une ou l'autre de ses formes énantiomériques selon que $R_1$ est respectivement un reste achiral ou chiral, la configuration absolue en position 4 de ce composé ainsi que celles qui en découlent en positions 1 et 5 étant alors déterminées par la stéréochimie du diastéréoisomère VII mis en œuvre, puis hydrolyse, si désiré, l'éther de formule V', en milieu acide, avec rétention

totale des configurations absolues, pour obtenir le composé de formule $V_A$ correspondant :

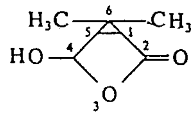

($V_A$)

Les exemples suivants illustrent l'invention sans toutefois la limiter.

Exemple 1

4-(2-nitro prop-2-yl)5-méthoxy tétrahydrofuran 2-one trans dl

On agite pendant 70 heures, à 20/25 °C, 9 $cm^3$ de 2-nitropropane 7 g de 5-méthoxy 2,5-dihydrofuran 2-one 3-ène et 1 $cm^3$ de triéthylamine, lave le mélange réactionnel avec une solution aqueuse de phosphate monosodique, extrait au benzène, sèche, concentre à sec sous pression réduite, reprend le résidu par de l'éther isopropylique, amorce la cristallisation et refroidit à 0 °C sous agitation, essore et obtient 8,42 g de produit cristallisé F ≃ 33 °C.

Analyse : $C_8H_{13}NO_5$ = 203,18
Calculé : C 47,29 H 6,45 N 6,89 %
Trouvé : C 47,10 H 6,50 N 6,70 %

Spectre de RMN

pic à 1,6 ppm attribué aux hydrogènes des méthyles géminés pics à 2,0-3,17 ppm attribués aux hydrogènes en 3 et 4 du cyclopentyle
pics à 5,26-5,28 ppm attribués à l'hydrogène en 5 du cyclopentyle
pic à 3,5 ppm attribué aux hydrogènes du méthoxy

Exemple II

4-(2-hydroxy prop-2-yl)5-(3-phénoxyphényl)méthoxy tétrahydrofuran-2-one-trans dl

On chauffe au reflux, sous agitation et sous atmosphère inerte, 3,5 g de 5-(3-phénoxyphényl)méthoxy 2,5-dihydrofuran-2-one dans 100 $cm^3$ d'isopropanol, ajoute régulièrement en une heure et demie par portions de 25 mg, 300 mg de peroxyde de benzoyle, concentre à sec sous pression réduite à 40-50 °C, chromatographie le résidu sur silice puis élue avec un mélange benzène-acétate d'éthyle 8-2, recueille 3,7 g de produit brut qui, repris par l'éther isopropylique, donne des cristaux blancs F = 50-55 °C.

Analyse : $C_{20}H_{22}O_3$ = 342,39
Calculé : C 70,16 H 6,4 %
Trouvé : C 69,9 H 6,5 %

Spectre de RMN (ppm)

pics à 1,18 et 1,21 attribués aux hydrogènes des méthyles
pics à 2,16-2,75 attribués aux hydrogènes en 3 et 4 du cyclopentyle
pics à 4,48-4,35 et 4,8-5 attribués aux hydrogènes du méthylène benzylique
pics à 5,59-5,58 attribués aux hydrogènes en 5 du cyclopentyle
pics à 6,83-7,5 attribués aux hydrogènes des noyaux aromatiques
pic à 1,5 attribué à l'hydrogène de l'hydroxyle

La 5-(3-phénoxyphényl) méthoxy 2,5-dihydrofuran-2-one utilisée au départ de l'exemple a été préparée comme suit : On mélange 12 g de 5 hydroxy 2-(5H) furanone, 250 $cm^3$ de benzène, 25 g d'alcool métaphénoxy benzylique et 200 mg d'acide paratoluène sulfonique, agite en distillant du benzène en le remplaçant plusieurs fois par la même quantité de benzène sec, dans le mélange réactionnel, après deux heures, laisse refroidir à température ambiante et lave avec une solution saturée de bicarbonate de sodium puis à l'eau, sèche, concentre à sec sous pression réduite, obtient 39,7 g d'une huile que l'on purifie par chromatographie sur silice en éluant par un mélange benzène-acétate d'éthyle 9-1 et recueille 24,9 g du produit attendu.

Spectre de RMN

pics à 7,3-7,4 ppm attribués aux hydrogènes en 4 de la furanone
pics à 6,18-6,32 ppm attribués aux hydrogènes en 3 de la furanone

pic à 6,03 ppm attribué à l'hydrogène en 5 de la furanone
pics à 4,58-4,76 et 4,85-5,12 ppm attribués aux hydrogènes du méthoxy
pics à 6,92-7,5 ppm attribués aux noyaux aromatiques

Exemple III

4-(2-paratoluène sulfonyl prop-2-yl)5-méthoxy tétrahydrofuran 2-one, trans dl

On mélange 500 mg d'isopropyl paratolyl sulfone et 10 $cm^3$ de tétrahydrofurane sous agitation et sous atmosphère inerte, refroidit à − 70 °C, ajoute lentement 1,3 $cm^3$ d'une solution cyclohexanique 1,95 M de butyl lithium, agite encore 1/2 heure à −70 °C, ajoute alors, en 10 minutes, 287 mg de 5-méthoxy 2,5-dihydrofuran 2-one en solution dans 4 $cm^3$ de tétrahydrofurane, maintient pendant une heure à − 70 °C, verse le mélange réactionnel sur une solution aqueuse de phosphate monosodique à 0 °C, extrait au chlorure de méthylène, lave à l'eau, sèche, concentre à sec sous pression réduite, cristallise le résidu dans l'éther isopropylique et obtient 490 mg de cristaux blancs F = 129 °C.

Analyse :

| | | | |
|---|---|---|---|
| Calculé : | C 57,67 | H 6,45 | S 10,26 % |
| Trouvé : | C 57,6 | H 6,5 | S 10,1 % |

Spectre de RMN

pics à 1,27-1,32 ppm attribués aux hydrogènes des méthyles géminés
pic à 2,46 ppm attribué aux hydrogènes du méthyle porté par l'aromatique
pic à 2,75 ppm attribué aux hydrogènes 3 et 4 du cyclopentyle
pic à 5,58 ppm attribué à l'hydrogène du cyclopentyle en 5
pic à 3,51 ppm attribué aux hydrogènes du méthoxy
pics à 7,28-7,42 ppm attribués aux hydrogènes en 3 et 5 de l'aromatique
pics à 7,66-7,8 ppm attribués aux hydrogènes en 2 et 6 de l'aromatique

Exemple IV

Iodure de [1(5-méthoxy 2-oxo 4,5-dihydro 3H furan-4-yl)-1-méthyl 1-éthyl] triphényl phosphonium

a) Préparation de l'ylure

A une suspension de 3 g d'iodure de triphényl isopropylphosphonium dans 40 $cm^3$ de tétrahydrofurane on ajoute en une fois 3,5 $cm^3$ de solution 2M de butyllithium dans le cyclohexane, agite pendant 10 minutes à température ambiante et obtient une solution d'ylure (solution A).

b) Addition de l'ylure sur la furanone

La solution A refroidie à − 60 °C est ajoutée lentement sous atmosphère inerte, à une solution de 0,800 g de 5-méthoxy 2,5-dihydrofuran 2-one 3-ène dans 50 $cm^3$ de tétrahydrofurane également refroidie à − 60 °C. On agite pendant 10 minutes, verse le mélange réactionnel sur un mélange de solution aqueuse de phosphate monosodique et de glace, lave à l'éther, extrait au chlorure de méthylène, sèche, concentre à sec sous pression réduite et obtient 3,4 g de sel de phosphonium, fondant vers 140 °C.

Analyse : $C_{26}H_8IO_3P$ = 546,37

| | | | | |
|---|---|---|---|---|
| Calculé : | C 57,15 | H 5,16 | I 23,24 | P 5,67 % |
| Trouvé : | C 57,3 | H 5,2 | I 22,4 | P 5,3 % |

Spectre de RMN

pics à 1,63-1,80-1,95-2,10 ppm attribués aux hydrogènes des méthyles en β du phosphore
pics à 2,22-3,33 ppm attribués aux hydrogènes portés par les carbones en position 3 et 4 de la furanone
pic à 3,23 ppm attribué aux hydrogènes du méthoxy
pics à 5,46-5,54 ppm attribués à l'hydrogène en 5 de la furanone
pics à 7,81-7,92 ppm attribués aux hydrogènes des noyaux aromatiques

Application 1

Acide 3-formyl 4-méthyl pent-3-èn 1-oïque

On dissout 18,9 g de carbonate de sodium dans 180 $cm^3$ d'eau, refroidit à 0 °C, ajoute, en une seule

fois, 18,2 g de la nitrolactone obtenue à l'exemple I et dissoute dans 25 $cm^3$ de méthanol. Après 120 heures à température ambiante, on lave le mélange réactionnel à l'éther éthylique, refroidit à 0 °C, ajoute avec précaution et sous atmosphère inerte, de l'acide sulfurique concentré jusqu'à pH ≃ 1, extrait au chloroforme puis à l'acétate d'éthyle, sèche les phases organiques réunies, les concentre à sec sous pression réduite, pour obtenir 9,5 g de produit brut qui, par cristallisation dans l'eau, donnent 7,4 g de produit pur F = 102 °C.

Analyse : $C_7H_{10}O_3$ = 142,156
Calculé : C 59,14 H 7,09 %
Trouvé : C 59,20 H 7,0 %

Spectre de RMN

pic à 20 ppm attribué aux hydrogènes en 5
pic à 2,26 ppm attribué aux hydrogènes du méthyle en 4
pic à 3,38 ppm attribué aux hydrogènes en 2
pic à 10,13 ppm attribué à l'hydrogène du formyle

Application 2

Acide 3-formyl 4-méthyl pent-3-èn 1-oïque

On dissout 619 mg de carbonate de sodium dans 6 $cm^3$ d'eau, refroidit à 0 °C, ajoute en agitant 1 g d'hydroxylactone obtenue à l'exemple 2 et 2 $cm^3$ de méthanol, après 19 heures de contact à température ambiante, lave à l'éther éthylique, refroidit à 0 °C, ajoute lentement de l'acide sulfurique concentré jusqu'à pH ~ 1, extrait au méthanol et au chloroforme, réunit les phases organiques, les sèche, les concentre à sec sous pression réduite, obtient 245 mg de produit brut qui, recristallisé, donne un produit fondant à 102 °C.

Application 3

Acide 3-formyl 4-méthyl pent-3-èn 1-oïque

On agite 250 mg du produit obtenu à l'exemple 3,4 $cm^3$ d'eau, 0,8 $cm^3$ de méthanol et 250 mg de carbonate de sodium pendant 2 jours à température ambiante. Après lavage à l'éther, on acidifie la phase aqueuse à pH-3,5, avec de l'acide chlorhydrique N, sature de chlorure de sodium, extrait au chloroforme, sèche, concentre à sec sous pression réduite, et obtient 87 mg du produit attendu F = 102 °C.

Ce produit est identique en tous points à l'acide 3-formyl 4-méthyl pent-3-èn 1-oïque obtenu par une autre voie.

Application 4

Acide 3-formyl 4-méthyl pent-3-ène 1-oïque

A une solution de 0,700 g d'iodure de [1(5-méthoxy 2-oxo 4,5-dihydro 3H furan-4-yl)-1-méthyl 1-éthyl] triphényl phosphonium dans 1 $cm^3$ de méthanol on ajoute 0,700 g de carbonate de sodium en solution dans 8 $cm^3$ d'eau, agite pendant 2 heures à 20 °C, élimine par filtration l'insoluble formé, acidifie le filtrat à pH 2, sature par du chlorure de sodium, extrait au chloroforme, concentre à sec, purifie dans l'éther isopropylique et obtient 0,095 g d'acide 3-formyl 4-méthyl pent-3-ène 1-oïque F = 102 °C.

Utilisation de l'acide 3-formyl 4-méthyl pent-3-ène 1-oïque issu de l'application

Utilisation 1

6,6-diméthyl 4-hydroxy 3-oxa bicyclo [3.1.0] hexan 2-one (dl)

Stade A

4-(2-chloro-prop-2-yl)5-hydroxy tétrahydrofuran 2-one dl trans

Sous un courant d'acide chlorhydrique anhydre, on agite 1 g d'acide 3-formyl 4-méthyl pent-3-ène 1-oïque obtenu à l'exemple 1, 2, 3 ou 4 $cm^3$ d'éther éthylique anhydre et 1 g de chlorure de lithium sec pendant 2 heures à – 30 °C, puis 2 heures à 0 °C, arrête le courant d'acide chlorhydrique et continue l'agitation 48 heures à température ambiante, après 54 heures de contact, verse le mélange réactionnel dans de l'eau glacée, décante, extrait au benzène, sèche, concentre à sec sous pression réduite, obtient

1,1 g de produit brut, le chromatographie sur silice en éluant par un mélange benzène-acétate d'éthyle 1-1, récupère 545 mg d'un produit qui cristallise F = 80 °C.

Analyse : $C_7H_{11}ClO_3$ = 178,617
Calculé : C 47,07 H 6,21 Cl 19,85 %
Trouvé : C 47,2 H 6,2 Cl 19,5 %

Spectre de RMN

pics à 1,55 et 1,66 ppm attribués aux hydrogènes des méthyles
pics à 2,42-2,92 ppm attribués aux hydrogènes en position 3 et 4 du cycle
pics à 5,82-5,89 ppm attribués à l'hydrogène en 5 du cycle
pic à 3,83 ppm attribué à l'hydrogène de l'hydroxyle

Stade B

4-(2-chloro-prop-2-yl)5-[(3-phénoxyphényl)méthoxy] tétrahydrofuran 2-one

On agite pendant 19 heures à température ambiante 393 mg du produit obtenu précédemment, 668 mg d'alcool métaphénoxybenzylique, 20 mg d'acide paratoluènesulfonique et 5 $cm^3$ de benzène, neutralise avec un peu de bicarbonate de sodium, sèche, concentre à sec sous pression réduite, obtient 1,18 g de produit brut, le chromatographie sur silice en éluant par du benzène, récupère 467 mg de produit, qui, recristallisé dans l'éther de pétrole, fond près de 50 °C.

Analyse : $C_{20}H_{21}ClO_4$ = 360,84
Calculé : C 66,57 H 5,87 Cl 9,83 %
Trouvé : C 66,60 H 5,80 Cl 9,90 %

Spectre de RMN

pics à 1,5 et 1,55 ppm attribués aux hydrogènes des méthyles
pics à 2,55-2,72 ppm attribués aux hydrogènes en position 3 et 4 du cyclopentyle
pics à 5,52-5,56 ppm attribués à l'hydrogène en position 5 du cyclopentyle
pics à 6,92-7,5 ppm attribués aux hydrogènes des noyaux aromatiques
pics à 4,47-4,66 et 4,77-4,97 ppm attribués aux hydrogènes du méthylène benzylique

Stade C

dl 6,6-diméthyl 4-hydroxy 3-oxabicyclo [3.1.0] hexan 2-one

On refroidit, vers − 20 °C, 0,55 $cm^3$ d'une solution molaire de diisopropylamine dans du tétrahydrofurane et 5 $cm^3$ de tétrahydrofurane, ajoute 0,25 $cm^3$ d'une solution de n-butyllithium 2M dans du cyclohexane, laisse remonter la température à 0 °C, puis refroidit à − 60 °C − 70 °C, ajoute en une seule fois 180 mg du produit obtenu au stade B, après 2 heures de réchauffement jusqu'à 0 °C, maintient 1 heure cette température, verse le mélange réactionnel dans de l'acide chlorhydrique 2N glacé et laisse 17 heures à 20 °C sous vive agitation, décante, extrait au chloroforme, sèche, concentre à sec, reprend le résidu obtenu par un mélange éther isopropylique-éther de pétrole, extrait à l'eau, concentre à sec sous pression réduite la phase aqueuse pour obtenir 30 mg de produit cristallisé F = 80 °C.

Utilisation 2

6,6-diméthyl 4-hydroxy 3-oxabicyclo [3.1.0] hexan 2-one (dl)

Stade A

4-(2-bromoprop-2-yl)5-hydroxy tétrahydrofuran 2-one dl trans

On refroidit à − 35 °C un mélange de 2,35 g d'acide 3-formyl 4-méthyl pent-3-ène 1-oïque obtenu à l'exemple 1 ou 2, 2,7 g de bromure de lithium sec et 60 $cm^3$ d'éther éthylique anhydre, agite 90 minutes sous courant d'acide bromhydrique sec, chasse celui-ci par un fort courant d'azote sec tout en maintenant la température entre − 30 °C et − 40 °C, verse le mélange réactionnel dans de l'eau glacée, extrait au benzène, sèche la phase organique, concentre à sec sous pression réduite sans chauffer, récupère 3,27 g d'une huile qui cristallise, recristallise le produit dans l'éther de pétrole et isole 2,5 g de cristaux blancs fondant à ~ 75 °C.

Analyse : $C_7H_{11}BrO_3$ = 223,073
Calculé : C 37,69 H 4,97 Br 35,82 %
Trouvé : C 37,80 H 5,20 Br 35,20 %

Spectre de RMN

pics à 1,74 et 1,86 ppm attribués aux hydrogènes des méthyles
pics à 2,25-3,0 ppm attribués aux hydrogènes en position 3 et 4 du cycle
pics à 5,87 et 5,93 ppm attribués à l'hydrogène en position 5 du cycle
pic à 3,92 ppm attribué à l'hydrogène de l'hydroxyle

Stabe B

4-(2-bromo-prop-2-yl)5 [(3-phénoxyphényl)méthoxy] tétrahydrofuran 2-one

On agite 24 heures à température ambiante 5,5 g du produit obtenu précédemment, 5,2 g d'alcool métaphénoxy benzylique, 50 $cm^3$ de benzène et 270 mg d'acide paratoluènesulfonique, lave le milieu réactionnel avec une solution aqueuse de bicarbonate de sodium, extrait au benzène, sèche, concentre à sec sous pression réduite, obtient environ 10 g d'une huile qui cristallise, recristallise ce produit dans un mélange d'éther isopropylique-éther de pétrole (7 $cm^3$-5 $cm^3$), isole 5,83 g fondant à 60 °C. Une chromatographie des liqueurs mères permet de récupérer 1 g de produit de même pureté.

Analyse : $C_{20}H_{21}BrO_4$
Calculé : C 59,27 H 5,22 Br 19,72 %
Trouvé : C 60,10 H 5,60 Br 21,50 %

Spectre de RMN

pics à 1,67-1,7 ppm attribués aux hydrogènes des méthyles
pics à 2,22-2,92 ppm attribués aux hydrogènes en position 3 et 4 du cyclopentyle
pics à 5,53-5,58 ppm attribués à l'hydrogène en position 5 du cyclopentyle
pics à 4,48-4,68 et 4,78-4,98 ppm attribués aux hydrogènes du méthylène benzylique
pics à 6,92-7,58 ppm attribués aux hydrogènes des noyaux aromatiques

Stade C

6,6-diméthyl 4-hydroxy 3-oxabicyclo [3.1.0] hexan 2-one

On refroidit, vers − 20 °C, 0,55 $cm^3$ d'une solution molaire de diisopropylamine dans du tétrahydrofurane diluée avec 5 $cm^3$ de tétrahydrofurane, ajoute 0,25 $cm^3$ d'une solution de n-butyllithium 2M dans le cyclohexane, laisse réchauffer le mélange vers 0 °C puis refroidit à − 60 °C pour ajouter en une seule fois 200 mg du dérivé bromé obtenu ci-dessus, après 15 minutes de réaction à cette température on obtient une solution de 6,6-diméthyl 4-[(3-phénoxyphényl)méthoxy] 3-oxabicyclo [3.1.0] hexan 2-one dl, la verse sur de l'acide chlorhydrique 2N glacé et laisse 17 heures à 20 °C sous vive agitation, décante, extrait au chloroforme, sèche, concentre à sec, obtient 170 mg de produit, le reprend par un mélange éther isopropylique-éther de pétrole, l'extrait à l'eau, concentre la phase aqueuse à sec sous pression réduite pour obtenir 50 mg de produit qui cristallise F = 80 °C.

## Revendications

1. Les composés de formule générale IV :

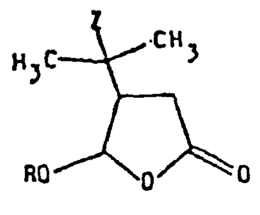

(IV)

dans laquelle Z représente un groupement électroattracteur et notamment un groupement hydroxy, un groupement nitro, un groupement arylsulfonyle ou un groupement halogéno triaryle phosphonio et R représente le reste d'un alcool R—OH.

2. Les composés de formule générale IV selon la revendication 1, caractérisés en ce que le groupement Z est un groupement $NO_2$.

3. Les composés de formule générale IV selon la revendication 1, caractérisés en ce que le groupement Z est un groupement hydroxyle.

4. Les composés de formule générale IV selon la revendication 1, caractérisés en ce que le groupement Z est un groupement arylesulfonyle.

5. Les composés de formule générale IV selon la revendication 1, caractérisés en ce que le groupement Z est un groupement halogénotriarylphosphonio.

6. Procédé de préparation des composés de formule générale IV, tels que définis à la revendication 1 caractérisé en ce que l'on fait réagir un dérivé de formule II :

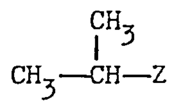

(II)

avec un dérivé de formule III :

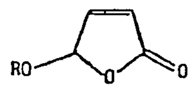

(III)

dans lesquelles R et Z ont les mêmes significations que dans la revendication 1, pour obtenir le composé de formule IV correspondant désiré.

7. Procédé selon la revendication 6, caractérisé en ce que le groupement Z est un groupement $NO_2$ et en ce que l'on fait réagir les composés II et III en présence d'une base choisie dans le groupe constitué par les amines secondaires, les amines tertiaires, les hydroxydes d'ammonium quaternaires et les carbonates alcalins.

8. Procédé selon la revendication 6, caractérisé en ce que le groupement Z est un groupement hydroxyle et en ce que l'on fait réagir les composés II et III sous irradiation ou en présence d'un promoteur radicalaire.

9. Procédé selon la revendication 6, caractérisé en ce que le groupement Z est un groupement aryl sulfonyle et en ce que l'on fait réagir les composés II et III en présence d'une base forte au sein d'un solvant organique.

10. Procédé selon la revendication 6, caractérisé en ce que le groupement Z est un groupement halogéno triaryl phosphonio et en ce que l'on fait réagir les composés II et III en présence d'une base forte au sein d'un solvant organique.

11. Procédé selon la revendication 9 ou 10, caractérisé en ce que la base forte est choisie dans le groupe constitué par les alkyl lithiens, les amidures alcalins, les hydrures alcalins, les alcoolates alcalins et en ce que le solvant utilisé est choisi dans le groupe constitué par le tétrahydrofurane, le diméthylsulfoxyde, le diméthoxyéthane, le diméthylformamide, l'hexaméthylphosphoramide, les hydrocarbures aromatiques et des cycloalcanes.

12. Application des composés de formule générale IV tels que définis à la revendication 1 à la préparation de l'acide 3-formyl 4-méthyl pent-3-èn 1-oïque caractérisée en ce que l'on soumet un composé de formule générale IV :

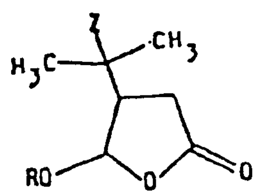

(IV)

dans laquelle R et Z conservent les significations de la revendication 1, à l'action d'un agent basique pour obtenir l'acide 3-formyl 4-méthyl pent-3-èn 1-oïque.

## Claims

1. The compounds with the general formula IV :

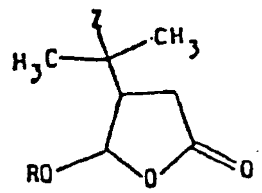

(IV)

in which Z represents an electro-attractor group and notably a hydroxy group, a nitro group, an arylsulphonyl group or a triaryl phosphonium halide group and R represents the residue of an alcohol R—OH.

2. The compounds with the general formula IV according to Claim 1, characterized in that the Z group is a group $NO_2$.

3. The compounds with the general formula IV according to Claim 1, characterized in that the Z group is a hydroxyl group.

4. The compounds with the general formula IV according to Claim 1, characterized in that the Z group is an arylsulphonyl group.

5. The compounds with the general formula IV according to Claim 1, characterized in that the Z group is a triaryl phosphonium halide group.

6. Preparation process for the compounds with the general formula IV, as defined in Claim 1, characterized in that a derivative with the formula II :

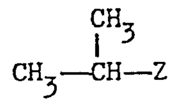

(II)

is made to react with a derivative with the formula III :

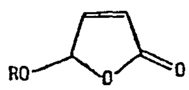

(III)

in which R and Z have the same significances as in Claim 1, so as to obtain the corresponding compound with the formula IV desired.

7. Process according to Claim 6, characterized in that the Z group is an $NO_2$ group and in that the compounds II and III are made to react in the presence of a base chosen from the group constituted by secondary amines, tertiary amines, quaternary ammonium hydroxides and alkaline carbonates.

8. Process according to Claim 6, characterized in that the Z group is a hydroxyl group and in that the compounds II and III are made to act under irradiation or in the presence of a radical promoter.

9. Process according to claim 6, characterized in that the Z group is an aryl sulphonyl group and in that the compounds II and III are made to react in the presence of a strong base in an organic solvent.

10. Process according to Claim 6, characterized in that the Z group is a triarylphosphonium halide group and in that the compounds II and III are made to react in the presence of a strong base in an organic solvent.

11. Process according to Claim 9 o 10, characterized in that the strong base is chosen from the group constituted by lithium alkyls, alkaline amides, alkaline hydrides, alkaline alcoholates and in that the solvent utilized is chosen from the group constituted by tetrahydrofuran, dimethylsulphoxide, dimethoxyethane, dimethylformamide, hexamethylphosphoramide, aromatic hydrocarbons and cyclo-alkanes.

12. Use of the compounds with the general formula IV as defined in Claim 1, in the preparation of 3-formyl-4-methyl-pent-3-en-1-oic acid, characterized in that a compound with the general formula IV :

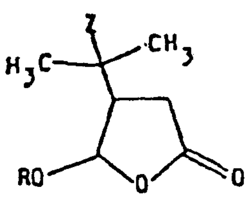

(IV)

in which R and Z retain the significances of Claim 1, is submitted to the action of a basic agent so as to obtain 3-formyl-4-methyl-pent-3-en-1-oic acid.

**Ansprüche**

1. Verbindungen der allgemeinen Formel IV

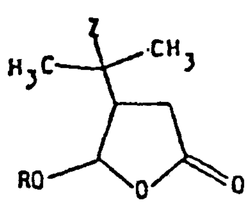

(IV)

worin Z eine elektronenanziehende Gruppe, und insbesondere eine Hydroxygruppe, eine Nitrogruppe, eine Arylsulfonylgruppe oder eine Halogenotriarylphosphoniogruppe bedeutet und R den Rest eines Alkohols R—OH darstellt.

2. Die Verbindungen der allgemeinen Formel IV gemäß Anspruch 1, dadurch gekennzeichnet, daß die Gruppe Z eine $NO_2$-Gruppe ist.

3. Die Verbindungen der allgemeinen Formel IV gemäß Anspruch 1, dadurch gekennzeichnet, daß die Gruppe Z eine Hydroxylgruppe ist.

4. Die Verbindungen der allgemeinen Formel IV gemäß Anspruch 1, dadurch gekennzeichnet, daß die Gruppe Z eine Arylsulfonylgruppe ist.

5. Die Verbindungen der allgemeinen Formel IV gemäß Anspruch 1, dadurch gekennzeichnet, daß die Gruppe Z eine Halogenotriarylphosphoniogruppe ist.

6. Verfahren zur Herstellung der Verbindungen der allgemeinen Formel IV gemäß Anspruch 1, dadurch gekennzeichnet, daß man ein Derivat der Formel II

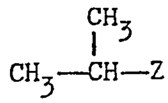

(II)

mit einem Derivat der Formel III

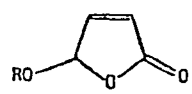

(III)

worin R und Z die in Anspruch 1 angegebenen Bedeutungen besitzen, umsetzt, um die entsprechende gewünschte Verbindung der Formel IV zu erhalten.

7. Verfahren gemäß Anspruch 6, dadurch gekennzeichnet, daß die Gruppe Z eine $NO_2$-Gruppe ist, und daß man die Verbindungen II und III in Gegenwart einer Base, ausgewählt unter den sekundären Aminen, den tertiären Aminen, den quaternären Ammoniumhydroxiden und den Alkalicarbonaten, umsetzt.

8. Verfahren gemäß Anspruch 6, dadurch gekennzeichnet, daß die Gruppe Z eine Hydroxylgruppe ist, und daß man die Verbindungen II und III unter Bestrahlung oder in Gegenwart eines radikalischen Promotors umsetzt.

9. Verfahren gemäß Anspruch 6, dadurch gekennzeichnet, daß die Gruppe Z eine Arylsulfonylgruppe ist, und daß man die Verbindungen II und III in Gegenwart einer starken Base in dem Medium eines organischen Lösungsmittels umsetzt.

10. Verfahren gemäß Anspruch 6, dadurch gekennzeichnet, daß die Gruppe Z eine Halogenotriarylphosphoniogruppe ist, und daß man die Verbindungen II und III in Gegenwart einer starken Base in dem Medium eines organischen Lösungsmittels umsetzt.

11. Verfahren gemäß Anspruch 9 oder 10, dadurch gekennzeichnet, daß die starke Base ausgewählt wird unter den Alkyllithiumverbindungen, den Alkaliamiden, den Alkalihydriden, den Alkalialkoholaten, und daß das verwendete Lösungsmittel ausgewählt wird unter Tetrahydrofuran, Dimethylsulfoxid, Dimethoxyäthan, Dimethylformamid, Hexamethylphosphoramid, den aromatischen Kohlenwasserstoffen und den Cycloalkanen.

12. Verwendung der Verbindungen der allgemeinen Formel IV gemäß Anspruch 1 zur Herstellung von 3-Formyl-4-methyl-pent-3-en-1-säure, dadurch gekennzeichnet, daß man eine Verbindung der allgemeinen Formel IV

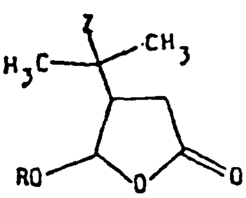

(IV)

worin R und Z die in Anspruch 1 angegebenen Bedeutungen besitzen, der Einwirkung eines basischen Mittels unterzieht, um die 3-Formyl-4-methyl-pent-3-en-1-säure zu erhalten.